# NEW EUROPEAN PATENT SPECIFICATION

(11) **EP 0 910 253 B2**
(45) Date of publication and mention of the opposition decision: **19.10.2011**
(45) Mention of the grant of the patent: 22.10.2003
(21) Application number: 98920738.6
(22) Date of filing: 01.05.1998
(51) Int. Cl.: A23L 1/30, A23L 1/305, A61K 31/195, A23L 1/09, A61K 31/70, A61K 35/20, A61K 31/715

(54) **PERI-OPERATIVE DRINK**
PERIOPERATIVES GETRÄNK
BOISSON PERI-OPERATOIRE

(30) Priority: 01.05.1997 EP 97201309
(43) Date of publication of application: 28.04.1999
(73) Proprietor: N.V. Nutricia, 2700 MA Zoetermeer (NL)
(72) Inventor: HOFMAN, Zandrie, NL-6721 RH Bennekom (NL); HAGEMAN, Robert, Johan, Joseph, NL-6705 CT WAGENINGEN (NL)
(74) Representative: van Westenbrugge, Andries
(86) International application number: PCT/NL1998/000242
(87) International publication number: WO 1998/049906

(56) References cited:
- EP-A- 0 527 283
- EP-A- 0 560 989
- WO-A-87/01589
- WO-A-91/18610
- WO-A-92/03155
- WO-A-92/10947
- DATABASE WPI Section Ch, Week 9038 Derwent Publications Ltd., London, GB; Class B05, AN 90-285845 XP002035110 & JP 02 200 165 A (SNOW BRAND MILK PROD CO LTD) , 8 August 1990

## Description

The invention pertains to a liquid nutritional composition for administration shortly before and after surgery and other conditions characterised by a demand for rapid gastric emptying and reversing catabolic processes and/or maintaining anabolism.

Before a medical operation, such as surgery, the patient is usually subjected to fasting for at least 8 hours, up to 24 hours, for reasons of safety with regard to anaesthesia and for preventing regurgitation of the stomach content and aspiration. This fasting, however, has undesired consequences for the patient. Firstly, the patient will suffer bad well-being as a result of hunger and thirst. Secondly, and more importantly, the patient's metabolism will be disturbed, and may even become catabolic. Such a catabolic state is highly undesired, since it results in loss of body weight and in energetically inefficient degradation of proteins, including proteins that are vital for local healing after the operation.

Therefore, there is a need for feeding the patient before surgery as long as possible, for example up to 90 minutes before operation, so as to keep the metabolism anabolic, without causing problems of anaesthesia and emptying of the stomach.

EP-A-564511 discloses a beverage for preoperative intake consisting of an aqueous solution which is hypotonic (250-295 mOsm/kg) and contains 8-20 g of carbohydrates per 100 ml. The carbohydrates consist of 10-30 % by weight of monosaccharides, 10-30 % by weight of disaccharides and the remainder (40-80 % by weight) polysaccharides (dextrins). No other ingredients are contemplated, except sodium or potassium chloride for adjusting the required osmolality and sodium hydroxide for adjusting the pH (5.6-6.8) and preservatives. The product was designed for rapidly passing the stomach and triggering hormonal response (insulin). It can be given to the patient until about 1 hour before operation.

EP-A-674902 discloses a dietary supplement to be given before operation, containing arginine or ornithine and ω-3 poly-unsaturated fatty acids (PUFA's), in addition to other ingredients including ω-6 PUFA's, proteins, carbohydrates, fats, nucleobases, salts, vitamins and the like. The product should stimulate the immune system after operation. It is not intended to be used shortly before operation, and the protein and fat content will ensure that the stomach residence time will be long.

Similarly, EP-A-704212 teaches the use of protected ω-3 PUFa's in a preoperative diet for minimising hepatic injury following surgery. Again, the product cannot be used shortly before surgery because of the presence of proteins and fats.

The preoperative use of arginine is also proposed in WO 96/36327. Its purpose is to improve micro-circulatory hypoperfusion. It is to be administered between 3 and 10 days before surgery.

WO 91/18610 discloses a solution for preoperative use containing glucose, potassium chloride and optionally glutamine or ornithine-α-ketoglutarate, fructose and insulin. The solution is administered parenterally and does not contain polysaccharides, fats, or other amino acids than glutamine.

WO 92/03155 discloses a composition containing glutamine, butyric acid and a growth factor having an anabolic effect on the intestinal mucosa. EP-A-560989 describes liquid enteral anticancer compositions containing amino acids and dextrin.

It was found that a liquid composition as defined in the claims, which is largely free of common fats and intact insoluble proteins, and contains soluble carbohydrates and glutamine, can be given to patients until shortly (e.g. 90 minutes) before operation, but also shortly after operation, without negative consequences for the patient during operation, and is effective in maintaining an anabolic metabolism and in particular in supporting protein synthesis and immune functions, so as to accelerate healing after the operation. The same composition was furthermore found to be suitable for feeding persons that require rapid gastric emptying and maintenance of an anabolic state, such as pregnant women just before delivery of the baby, persons that are in a state of high physical exercise, for example during sports and during certain phases in cancer treatment.

The composition according to the invention is defined in the appending claims.

The composition contains 24-80 g of soluble carbohydrates per 400 ml. The soluble carbohydrates preferably contain a majority of polysaccharides (i.e. more than 50 % by weight). In particular the weight percentage of polysaccharides is at least 75 % or even at least 80 %. The level of soluble polysaccharides is at least 3.75 g per 400 ml. The polysaccharides are meant to be any water-soluble, preferably digestible, saccharides containing more than two monosaccharide units and may be constituted by dextrins, maltodextrins and the like. Preferably, the polysaccharide also comprises longer chains e.g. maltodextrin molecules having more than 10 monosaccharide units. A preferred monosaccharide is fructose, as it induces a delayed insulin response, and does not impact gastric emptying. Fructose is preferably present at a level of 1-10 g per 400 ml. Preferably no substantial amounts of other mono- and disaccharides are present in the composition; in particular the glucose content is less than 5 g per 400 ml.

Glutamine is present at a level of 1-30 g per 400 ml, preferably from 5 to 25 g / 400 ml. It may be present as such, but it may also be present as a precursor of glutamine, like glutamine-rich peptides, such as glutaminylglycine, glutamylglutamine or alanyl-glutamine or mixtures thereof or other glutamine-containing sources. Also water-soluble hydrolysates of glutamin-rich proteins (like wheat-gluten protein) can be used as an ingredient, if they are low in salt content (ash). Demineralisation preferably occurs before spray-drying using known methods. In case of a glutamine precursor, its level is based on its glutamine content. A protein hydrolysate containing 20-35 wt.% of glutamine (as only glutamine source), such as for example derived from wheat gluten, can be used at a level of 5-70 g per 400 ml. The composition contains 0.2-8 g of methionine and/or cysteine and/or contains 2-8 g of arginine.

It is preferred that the composition also contains 0.5-20, especially 2-15 g / 400 ml of other amino acids such as arginine, ornithine, lysine or histidine, or their metabolic precursors. Preferably these amino acids are included at a level of 0.5-15 g, especially 2-8 g / 400 ml. Among these, arginine is especially preferred.

The composition according to the invention advantageously also contains growth factors, e.g. derived from a liquid dairy product containing these. Preferably colostrum or a fraction thereof is used. The liquid dairy product may represent 1-100 ml per 400 ml of the composition. It can be used e.g. as a spray-dried defatted colostrum whey at a rate of 0.1-10 g whey powder. If a growth factor isolate from colostrum is used, 1-1000 mg thereof can be used.

The composition also comprises 0.2-8 g, especially 0.3-3.0 g of methionine and/or cysteine or their metabolic equivalents (such as N-acetylcysteine) per 400 ml. Further desirable amino acids comprise glycine, serine, alanine and glutamate, which may each be present at 0.2-20 especially 0.5-8 g / 400 ml. The composition contains 0.2-8 g of Methionine and/or Cysteine, and/or contains 2-8 g of Arginine.

Furthermore the composition may contain vitamins, trace elements, minerals and other components, for example those given below or their metabolic equivalents.

| | *preferred amount per 400 g* |
|---|---|
| * Trace elements, minerals | |
| magnesium | 60-400 mg |
| zinc | 4-40 mg |
| copper | 0.6-20 mg |
| selenium | 20-300 µg |
| manganese | 2-40 mg |
| chromium | 20-400 µg |

| * Vitamins | |
|---|---|
| folic acid | 80-1000 µg |
| pyridoxine | 0.7-18 mg |
| cyanocobalamine | 0.8-16 µg |
| vitamin C | 20-400 mg |
| vitamin D | 0.5-20 µg |
| thiamin | 0.4-6 mg |
| riboflavin | 0.5-7 mg |
| nicotinamide | 5-75 mg NE |
| biotin | 30-400 µg |

| * Other components | |
|---|---|
| nucleotides | 2-500 mg yeast extract |
| lecithin or other phospholipids, medium-chain triglyderides (MCT's) creatine, pyruvate, carotenoids, flavonoids. | |

Also a blend of antioxidants like selenium, ascorbic acid, tocopherols, carotenoids and flavonols can be included advantageously. Preferably the composition is low in fats and proteins. In particular it contains less than 2 % by weight of fat. The fat preferably contains less 2 %, more preferably less than 1 % by weight of lauric and myristic fatty acids with respect to the total fatty acids. Especially if the fat is present at a level of more than 0.5 %, it advantageously consists of at least 50 % by weight of medium-chain fatty acids (C₈ and C₁₀), lecithins, phospholipids or structured fats. The fat further preferably contains 3-60 wt.% of long-chain polyunsaturated fatty acids. The preferred fat level is 0.3-1.5 wt.%.

The composition contains 0.5-4 wt.% of soluble whey proteins, preferably 0.5-2 wt.% (soluble is understood to mean soluble at pH 2.0 to 7.5). It contains less than 0.5 wt.%, more preferably less than 0.1 wt.% of insoluble at pH 3 intact proteins, such as caseins. The osmolality should not be too high although hypotonicity is not absolutely necessary. The osmolality may e.g. be between 250 and 450, especially between 300 and 400 mOsm/kg. The pH of the composition is between 4.0 and 8.0, preferably between 5.5 and 7.5. If necessary the desired can be adjusted by addition of a buffering agent such as phosphate or especially citrate. The energy density of the composition is not more than 1.3, and preferably less than 1.0 kcal per ml of (reconstituted) liquid.

The composition is a liquid composition which can be administered as such. The composition may be prepared by methods known per se, which may comprise dissolving, mixing, buffering, homogenising, pasteurising, spray-drying, and the like. It can be administered enterally e.g. by drinking or tube-feeding. It can be administered in an amount of 50 to 600 ml - depending on the patient - up to 1 hour before operation, if appropriate preceded by similar or higher amounts longer before the operation. From half an hour onwards after the operation, similar amounts can be given according to the needs of the patient. For other conditions, such as delivery or other physical exertion, adapted amounts can be given. On average, the amount to be administered is preferably 400 ml per day.

### Example 1 [not according to the invention]

A liquid perioperative composition was prepared by mixing the following components:

| *Ingredient:* | *Amount per 5000 l* |
|---|---|
| Maltodextrin DE19 | 625 kg |
| Wheat protein hydrolysate | 375 kg |
| L-Arginine | 37.5 kg |
| L-Lysine.HCl | 25 kg |
| L-Methionine | 6.25 kg |
| Bovine colostrum whey protein powder | 7.5 kg |
| Ascorbic acid | 625 g |
| Pyridoxamine | 25 g |
| Folic acid | 2.5 g |
| Niacin | 250 g NE |
| Biotin | 1.25 g |
| Trimagnesium dicitrate | 4.4 kg |

The energy density of this composition was about 0.86 kcal/ml. The glutamine content was about 9 g / 400 ml and the pH was 6.7.

### Example 2 [not according to the invention]

A liquid perioperative composition was prepared by mixing the following components:

| *Ingredient:* | *Amount per 5000 l* |
|---|---|
| Maltodextrin DE19 | 710 kg |
| Fructose | 40 kg |
| Alanylglutamine | 310 kg |

The energy density of this composition was about 0.85 kcal/ml.

## Claims

1. A liquid nutritional composition suitable for enteral peri-operative use, containing, per 400 ml, 24-80 g of soluble carbohydrates, at least 3.75 g of which are polysaccharides, less than 2 wt.% d fat and less than 0.5 wt.% of intact proteins that are insoluble at pH 3, the composition containing 1-30 g of glutamine or a glutamine precursor calculated as glutamine, and furthermore containing 0.2-8 g of methionine and/or cysteine, and/or containing 2-8 g of arginine, and containing 0.5-4 wt.% of whey proteins being soluble at ph 2.0 to 7.5.

2. A composition according to claim 1, the carbohydrates consisting of at least 75% by weight of polysaccharides, and optionally 1-10 g of fructose.

3. A composition according to any one of claims 1-2, further containing 0.5-20 g of one or more amino acids selected from arginine, lysine, ornithine and histidine per 400 ml.

4. A composition according to any one of claims 1-3, further containing 0.2-20 g of each of glycine, alanine, serine and/or glutamate per 400 ml.

5. A composition according to any one of claim. 1 to 4, containing 5-25 g of glutamine or glutamine precursor per 400 ml.

6. A composition according to any one of claims 1-5 further containing, per 400 ml, 1-100 ml of liquid dairy product rich in growth factors, especially containing defatted colostrums corresponding to 0.1-10 g of whey protein powder.

7. A composition according to any one of claims 1-6, having a fat content of 0.3-1.5 % by weight of the total composition, the fat preferably comprising less than 1 % of C₁₂ and C₁₄ fatty acids by weight of the total fatty acids.

8. A composition according to any one of claims 1-7, having an osmolality between 250 and 450 mOsm/kg, and an energy density below 1.0 kcal/ml.

9. A process for preparing a liquid composition suitable for enteral peri-operative use, comprising adding water to the dry ingredients according to any one of claims 1-8.

## Patentansprüche

1. Flüssige Ernährungszusammensetzung, geeignet zur enteralen perioperativen Verwendung, enthaltend pro 400 ml 24-80 g lösliche Kohlenhydrate, von welchen mindestens 3,75 g Polysaccharide sind, weniger als 2 Gewichtsprozent (Gew.-%) Fett und weniger als 0,5 Gew.-% intakte Proteine, die bei pH 3 unlöslich sind, wobei die Zusammensetzung 1-30 g Glutamin oder eine Glutaminvorstufe, berechnet als Glutamin, enthält und außerdem 0,2-8 g Methionin und/oder Cystein enthält und/oder 2-8 g Arginin enthält und 0,5-4 Gew.% Molkeproteine, die bei pH 2,0 bis 7,5 löslich sind.

2. Zusammensetzung gemäß Anspruch 1,
wobei die Kohlenhydrate vorzugsweise aus mindestens 75 Gew.-% Polysaccharide und gegebenenfalls 1 -10 g Fructose bestehen.

3. Zusammensetzung gemäß einem der Ansprüche 1-2, die ferner 0,5-20 g einer oder mehrerer Aminosäuren, ausgewählt aus Arginin, Lysin, Ornithin und Histidin, pro 400 ml enthält.

4. Zusammensetzung gemäß einem der Ansprüche 1-3, die ferner jeweils 0,2-20 g Glycin, Alanin, Serin und/oder Glutamat pro 400 ml enthält.

5. Zusammensetzung gemäß einem der Ansprüche 1-4, die 5-25 g Glutamin oder Glutaminvorstufe pro 400 ml enthält.

6. Zusammensetzung gemäß einem der Ansprüche 1-5, die ferner pro 400 ml 1-100 ml flüssiges Molkereiprodukt enthält, das reich an Wachstumsfaktoren ist, die besonders entfettetes Kolostrum enthält, das 0, 1 -10 g Molkeproteinpulver entspricht.

7. Zusammensetzung gemäß einem der Ansprüche 1-6, die einen Fettgehalt von 0,3-1,5 Gew.-% der Gesamtzusammensetzung aufweist, wobei das Fett vorzugsweise weniger als 1 Gew.-% C₁₂ und C₁₄ Fettsäuren der Gesamtfettsäuten umfasst.

8. Zusammensetzung gemäß einem der Ansprüche 1-7, die eine Osmolalität zwischen 250 und 450 mOsm/kg und eine Energiedichte unter 1,0 kcal/ml aufweist.

9. Verfahren zur Herstellung einer flüssigen Zusammensetzung, geeignet zur enteralen perioperativen Verwendung, umfassend das Hinzufügen von. Wasser zu den trockenen Bestandteilen gemäß einem der Ansprüche 1-8.

## Revendications

1. Composition nutritionnelle liquide adaptée à une utilisation péri-opératoire par voie entérale, contenant, pour 400 ml, 24 à 80 g de glucides solubles dont au moins 3,75 g sont des polysaccharides, moins de 2 % en poids de graisse et moins de 0,5 % en poids de protéines intactes qui sont insolubles à pH 3, la composition contenant 1 à 30 g de glutamine ou d'un précurseur de la glutamine calculé en glutamine, et contenant en outre 0,2 à 8 g de méthionine et/ou de cystéine, et/ou contenant 2 à 8 g d'arginine et contenant 0,5 à 4 % en poids de protéines de lactosérum qui sont solubles de pH 2,0 à 7,5.

2. Composition selon la revendication 1, les glucides consistant en au moins 75 % en poids de polysaccharides et facultativement, 1 à 10 g de fructose.

3. Composition selon l'une quelconque des revendications 1 à 2, contenant en outre 0,5 à 20 g d'un ou plusieurs acides aminés choisis parmi l'arginine, la lysine, l'ornithine et l'histidine pour 400 ml.

4. Composition selon l'une quelconque des revendications 1 à 3, contenant en outre 0,2 à 20 g de chacun des composés glycine, alanine, sérine et/ou glutamate pour 400 ml.

5. Composition selon l'une quelconque des revendications 1 à 4, contenant 5 à 25 g de glutamine ou de précurseur de glutamine pour 400 ml.

6. Composition selon l'une quelconque des revendications 1 à 5, contenant en outre pour 400 ml, 1 à 100 ml de produit laitier liquide riche en facteurs de croissance, contenant spécialement du colostrum dégraissé correspondant à 0,1 à 10 g de protéines de lactosérum en poudre.

7. Composition selon l'une quelconque des revendications 1 à 6, ayant une teneur en graisse de 0,3 à 1,5 % en poids de la composition totale, la graisse comprenant de préférence moins de 1 % d'acides gras en C₁₂ et C₁₄ en poids du total des acides gras.

8. Composition selon l'une quelconque des revendications 1 à 7, ayant une osmolalité entre 250 et 450 mOsm/kg, et une densité d'énergie inférieure à 1,0 kcal/ml.

9. Procédé de préparation d'une composition liquide adaptée à une utilisation péri-opératoire par voie entérale, comprenant l'ajout d'eau aux ingrédients secs selon l'une quelconque des revendications 1 à 8.
